# EUROPEAN PATENT SPECIFICATION

(11) **EP 1 021 565 B1**
(45) Date of publication and mention of the grant of the patent: **31.07.2002**
(21) Application number: 98967096.3
(22) Date of filing: 02.01.1998
(51) Int. Cl.: C12Q 1/68

(54) **Z-CHROMOSOMAL MARKERS DERIVED FROM CHICKEN (GALLUS DOMESTICUS) AND USE THEREOF IN CHROMOSOMAL MAPPING**
Z-CHROMOSOMALE MARKER VON HUHN (Gallus domesticus) UND DEREN VERWENDUNG ZUR CHROMOSOMALEN KARTIERUNG
MARQUEURS TIRES DE CHROMOSOMES Z DERIVES DU POULET (GALLUS DOMESTICUS) ET LEUR UTILISATION DANS LA CARTOGRAPHIE CHROMOSOMIQUE

(30) Priority: 02.01.1997 US 34410 P
(43) Date of publication of application: 26.07.2000
(73) Proprietor: University of Massachusetts, a Public Institution of Higher Education of The Commonwealth of Massachusetts,, Amherst, MA 01002 (US)
(72) Inventor: Ponce de Leon, F. Abel, Amherst, MA 10002 (US); Ciufo, Stacy, Amherst, MA 01002 (US); Robl, James, Belchertown, MA 01007 (US); Ambady, Sakthikumar, Kerala State (IN); Smyth, J. Robert, Jr., Amherst, MA 01002 (US)
(74) Representative: Goldin, Douglas Michael
(86) International application number: US9808896
(87) International publication number: WO9837243

(56) References cited:
- WO-A-94/07907
- WO-A-96/39505
- LEVIN I ET AL: "Genetic map of the chicken Z chromosome using random amplified polymorphic DNA (RAPD) markers." GENOMICS, (1993 APR) 16 (1) 224-30, XP002067078 cited in the application
- BRUFORD M W ET AL: "Minisatellite DNA markers in the chicken genome. II. Isolation and characterization of minisatellite loci." ANIMAL GENETICS, (1994 DEC) 25 (6) 391-9, XP002067079
- BIOLOGICAL ABSTRACTS, vol. 95, Philadelphia, PA, US; abstract no. 423269, PONCE DE LEON F A ET AL: "Analysis of the chicken NM7659 T( Z;1) translocation with chromosome painting probes and GBP banding." XP002067083 & EIGHTY-FOURTH ANNUAL MEETING OF THE POULTRY SCIENCE ASSOCIATION, INC., EDMONTON, ALBERTA, CANADA, AUGUST 14-18, 1995. POULTRY SCIENCE 74 (SUPPL. 1). 1995. 9. ISSN: 0032-5791,
- BIOLOGICAL ABSTRACTS, vol. 95, Philadelphia, PA, US; abstract no. 423268, AMBADY S ET AL: "A Z - chromosome specific DNA library." XP002067084 & EIGHTY-FOURTH ANNUAL MEETING OF THE POULTRY SCIENCE ASSOCIATION, INC., EDMONTON, ALBERTA, CANADA, AUGUST 14-18, 1995. POULTRY SCIENCE 74 (SUPPL. 1). 1995. 8. ISSN: 0032-5791,
- PONCE DE LE N ET AL.: "Development of a bovine X chromosome linkage group and painting probes to asses cattle, sheep and goat X chromosome segment homologies" PROCEEDINGS OF THE NATIONAL ACADEMY OF SCIENCES OF USA, vol. 93, April 1996, WASHINGTON US, pages 3450-3454, XP002067080 cited in the application
- AMBADY S ET AL: "Development of a chicken Z - chromosome -specific DNA library." JOURNAL OF HEREDITY, (1997 MAY-JUN) 88 (3) 247-9, XP002067081
- ZIMMER R ET AL: "Generation of chicken Z - chromosome painting probes by microdissection for screening large-insert genomic libraries." CYTOGENETICS AND CELL GENETICS, (1997) 78 (2) 124-30, XP002067082
- BIOLOGICAL ABSTRACTS, vol. 97, Philadelphia, PA, US; abstract no. 487182, PONCE DE LEON F A ET AL: "Chicken genome project: Chromosome-specific libraries and applications of genome scans to assess genomic variation." XP002067085 & REVISTA BRASILEIRA DE REPRODUCAO ANIMAL 21 (3). 1997. 102-105. ISSN: 0102-0803,

## Description

### Field of the Invention

The invention relates to novel chromosomal markers derived from chicken and use thereof.

### Background of the Invention

Livestock genome maps have progressed very rapidly in the past few years due to the availability of highly polymorphic DNA markers. But in many species, the maps are not dense enough to facilitate a thorough search for quantitative trait loci (QTLs). This is especially true in the case of the chicken. The chicken haploid karyotype consists of 39 chromosomes that are classified into two categories - the macrochromosomes and the microchromosomes. The largest five pairs of macrochromosomes and the Z-chromosome represent about 55 percent of the total DNA content of the chicken genome. The Z-chromosome covers about 210 cM of the estimated 2500 - 3,000 cM of the chicken genome map (Levin et al. *Genomics,* 16:224-230 (1993)).

Knowledge of the genetic composition of the chicken Z-chromosome is limited, in spite of the fact that this chromosome has the most detailed linkage map for this species, largely generated by classical linkage test analyses (Bitgood and Somes, *Poultry Breeding and Genetics*, 2nd Ed., Crawford RD, ed., Amsterdam: Elsevier, pp. 469-495 (1990)). To date, 19 known loci and 14 genetic markers consisting of 3 chicken middle repetitive sequence element (CRI) markers, 8 random amplified polymorphic DNA (RAPD) markers and 3 microsatellites have been assigned to the chicken Z-chromosome (Bitgood and Somes, (Id.) (1990); Saitoh et al, *Chrom. Res.*, 1: 239-251 (1993); Cheng et al, *Poultry Sci.*, 74: 1855-1874 (1995)).

The avian sex chromosome constitution differs from that of mammals because females are heterogametic (ZW) and males homogametic (ZZ). It has been observed from comparative linkage analyses that some of the sex linked genes in mammals are autosomal in chicken, while some of the sex linked genes in chicken are autosomal in mammals (Bitgood and Somes, (Id.) (1990)). Accordingly, obtaining further information concerning the Z-chromosome of chickens would be beneficial in identifying sex-linked genes in chickens and related species.

### Brief Description and Obiects of the Invention

Thus, it is an object of the invention to identify novel chromosomal markers from the Z-chromosome of chicken. It is further an object of the invention to use such markers to construct a Z-chromosome specific DNA map and to use such chromosomal markers to identify Z-chromosome homologs in related avian species, e.g., turkey.

In order to develop a dense genetic map for chicken, it is important to generate a large number of polymorphic markers per chromosome (Cheng et al, *Poultry Sci.,* 741:1855-1874 (1995)). One way of achieving this goal is to develop chromosome-specific libraries. Chromosome flow-sorting has been the method of choice for the generation of chromosome-specific libraries in humans (Fuscoe et al, *Cytogenet Cell Genet,* 43:79-86 (1986)) and in swine (Langford et al, *Anim. Genet, 24:* 261-267 (1993)). Development of flow-sorted chromosomes is technically demanding and frequently yield preparations which have some degree of contamination with other chromosomes (Hozier and Davis, *Anal. Biochem,* 200: 205-127 (1992)).

A more effective and direct way of generating chromosome-specific DNA libraries is by chromosome microisolation and microcloning of the chromosome of interest. Chromosome specific libraries generated by chromosome microisolation have been used in swine (Ambady et al, (unpublished data)), cattle (Ponce de León et al, *Proc. Natl. Acad. Sci., USA*, (in press) 1996)), and chicken (Li et al, *Proc. of the 10th Eur. Colloq. on Cytogenetics of Domestic Animals*, Utrecht Univ., The Neth., p. 11, August 18-21 (1992)) genetic mapping studies in order to develop maps for particular chromosomes. Generation of polymorphic markers from chromosome-specific libraries for all of the 8 pairs of the chicken macrochromosomes will enable saturation of about 55-70% of the chicken genome. Chromosome-specific DNA can also be used as heterologous chromosome painting probes in closely and distantly related species for comparative genome analysis, study of chromosomal evolution, and for identifying gross chromosomal abnormalities.

This application, in particular, provides a Z-chromosomal marker DNA selected from the group consisting of Sequence I (43. Seq), Sequence 2 (71. Seq), Sequence 3 (80. Seq), Sequence 4 (81. Seq), Sequence 5 (131. Seq), Sequence 6 (147. Seq), Sequence 7 (166. Seq), Sequence 8 (196. Seq), Sequence 9 (199. Seq), Sequence 10 (204. Seq), Sequence 11 (235. Seq), Sequence 12 (249. Seq), Sequence 13 (258. Seq), Sequence 14 (290. Seq), Sequence 15 (309. Seq), Sequence 16 (341. Seq), Sequence 17 (398. Seq), Sequence 18 (420. Seq), and Sequence 19 (435. Seq).

The invention also provides a Z-chromosomal DNA library that contains at least one marker DNA sequence of the invention and methods using at least one marker DNA sequence of the invention for genetic mapping.

### Brief Description of the Figures

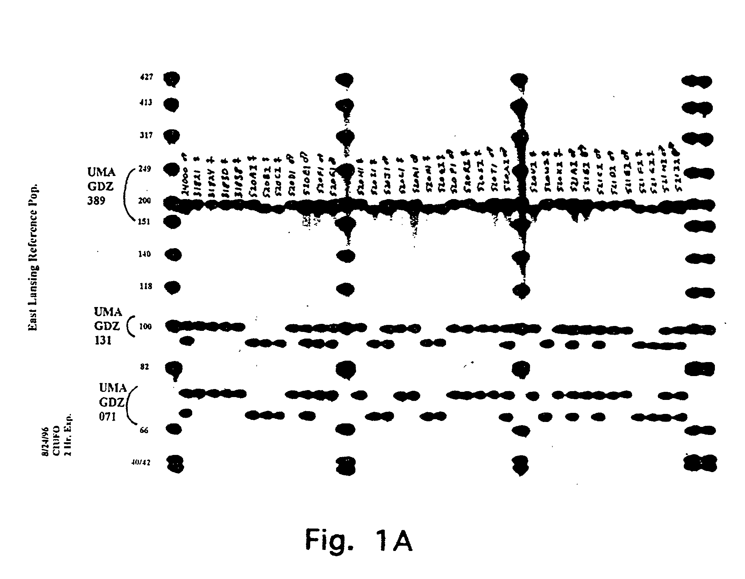

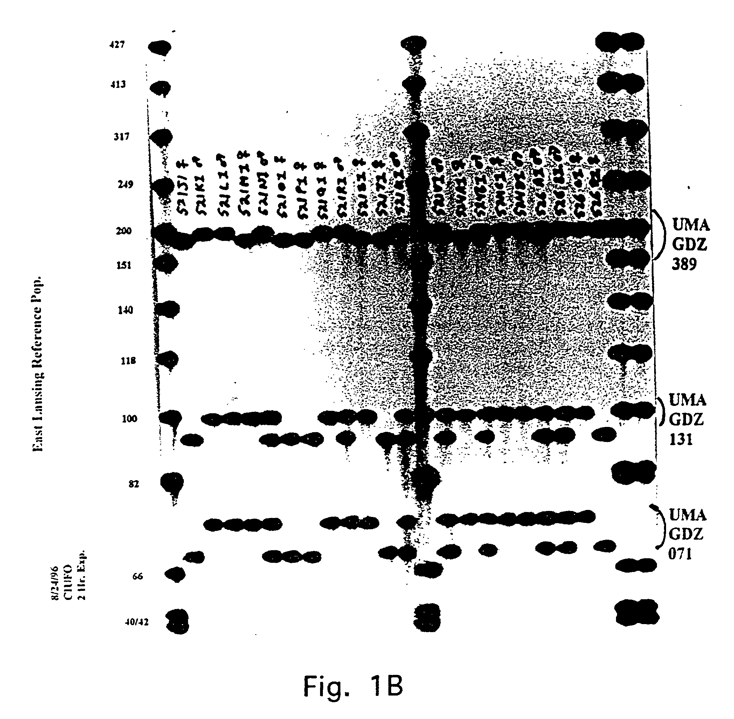

Figure 1 shows amplification of microsatellite markers by PCR and identification of polymorphisms.

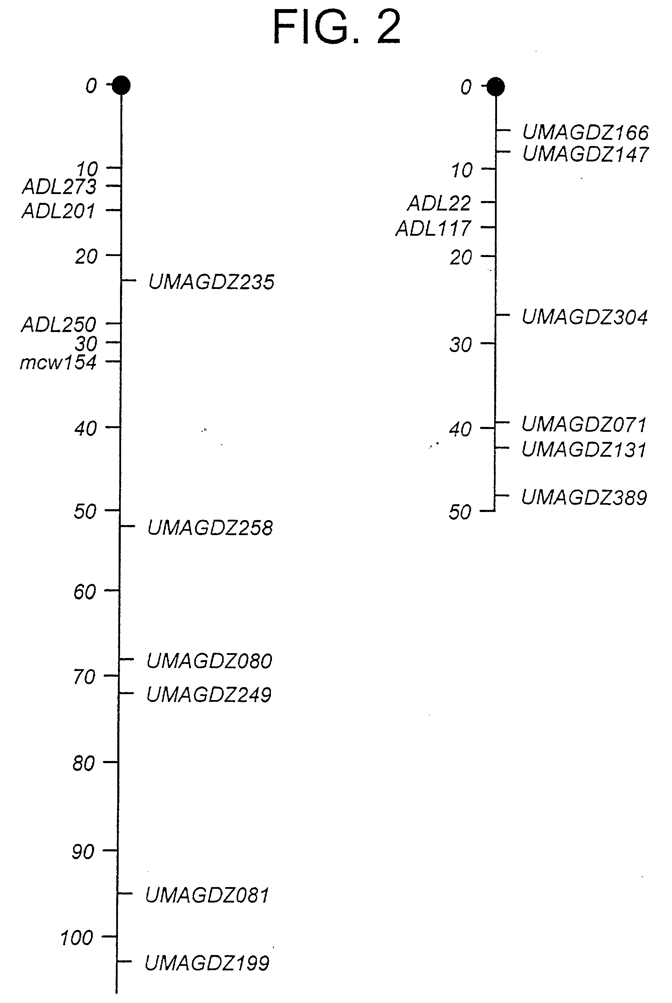

Figure 2 shows a genetic map constructed using the identified microsatellite markers.

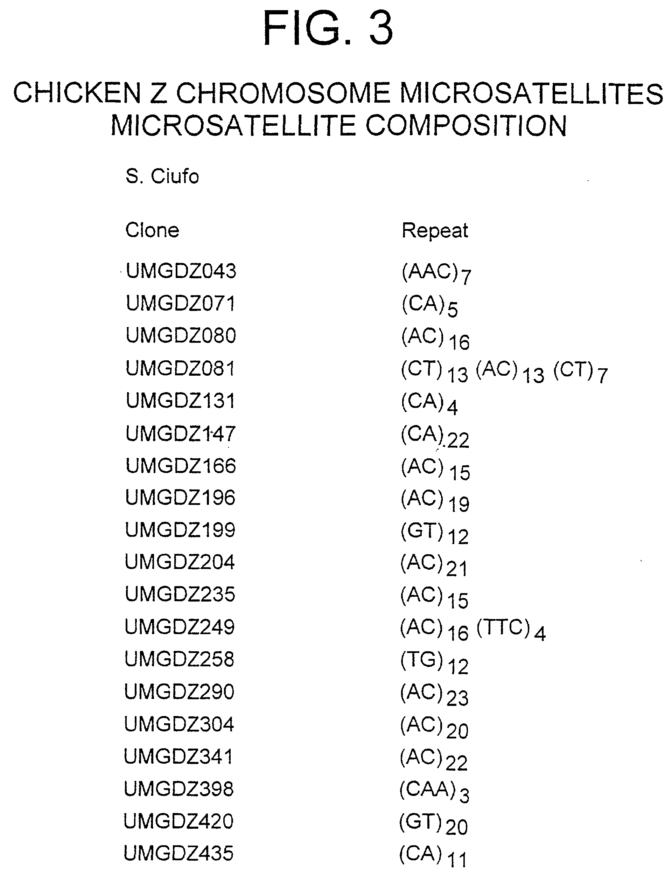

Figure 3 shows dinucleotide repeats present in the identified microsatellite markers.

### Detailed Description of the Invention

### Microisolation and microcloning:

Chicken metaphases were prepared from chicken fibroblast cultures following standard procedures, fixed briefly for 5 minutes each in 9:1, 5:1 and 3:1 methanol:acetic acid and dropped on clean coverslips. Chromosome microisolation and cloning was performed following the procedure described by Ponce de León et al (*Proc. Natl. Acad. Sci., USA* (in press) (1996)). Briefly, twelve copies of the chicken Z-chromosome were microisolated and transferred to clean siliconized coverslips. Proteinase-K digestion, phenol-chloroform extraction, *Sau*3AI (50U/µl, New England Biolabs) digestion and ligation to custom prepared *Sau*3AI adaptors were performed in a nanoliter drop. Ligation products were digested with BgII enzyme (Promega, 10 units/µl) to cleave off the adaptor dimers that form during the ligation process.

The ligation product was PCR amplified and 10 µl of the amplified product was run on an agarose gel to determine the size of the amplified products. A 2 µl volume of this original amplification was labeled by PCR, using biotin-16-dUTP (Boehringer Mannheim). The purity, specificity and origin of the DNA fragments was determined by FISH on chicken metaphases following the procedure described by Ponce de León et al (*Proc. Natl. Acad. Sci. USA* (in press) (1996)). The remainder of the PCR product was digested with *Sau*3AI and passed through a Microcon 30 (Amicon Inc.) spin column to cleave and remove the flanking adaptors respectively.

In order to produce a chicken Z-chromosome-specific phage library, the digested DNA was cloned in a lambda ZAP Express vector (Stratagene) and packaged using Gigapack II Gold packaging extract (Stratagene). The library was amplified by plate lysate method following the manufacturer's protocol and stored at -70°C in 7% DMSO and 0.3% chloroform. Average size of library inserts was determined by PCR amplification of 30 randomly picked clones using the T3 and T7 priming sites flanking the insert.

### Fluorescent in situ hybridizations

The Z-chromosome-specific DNA fragments were fluorescently labeled by PCR with biotin-16-dUTP (3:1 ratio of dTTP:biotin-16-dUTP) and passed through a Sephadex G-50 column to remove unincorporated nucleotides. The protocol described by Ponce de León (*Proc. Natl. Acad. Sci., USA* (in press) (1996)) was followed. Briefly, 200 nanograms of labeled Z-chromosome specific DNA was mixed with 6 µg of chicken competitor DNA (average size 200-400 bp) and 5.8 µg of salmon sperm DNA (average size 200-400 bp), precipitated and resuspended in 12 µl of hybridization buffer consisting of 50% deionized formamide, 1X SSC and 100% dextran sulphate to achieve a final DNA concentration of 1 µg/µl. The hybridization mix was denatured at 75°C for 5 minutes and reannealed at 37°C for 10 minutes and deposited on denatured (70% formamide, 2X SSC at 70°C for 2 minutes) chicken or turkey metaphases, mounted, sealed with rubber cement and incubated in a humidified chamber at 37°C for 18 to 20 hours. The slides were washed in 50% formamide/2X SSC at 42°C for 15 minutes and 0.1X SSC at 60°C for 15 minutes. Blocking was done using 2% blocking reagent (Boehringer Mannheim) and the signals were detected using avidin-FITC (5 µg/ml, Vector labs) in 1% blocking solution. Slides were washed in 4X SSC/0.1% Tween-20 for 15 minutes at 42°C, stained for 10 minutes in propidium iodide (400 ng/ml in 2X SSC) and rinsed for 5 minutes in 2X SSC/0.01% Tween-20. Slides were mounted in p-phenylenediamine-11 (PPD-11) antifade and observed under a Zeiss Axioskop fluorescent microscope.

### Results

A chicken Z-chromosome specific DNA cocktail was developed by chromosome microisolation, *Sau*3AI digestion, adaptor ligation and PCR amplification. The amplified DNA fragments ranged in size from 400 bp to 1600 bp with the bulk of the DNA in the 500-1000 bp range. The origin, specificity and purity of the chromosomal DNA fragments was verified by FISH after PCR labeling of a small fraction of the DNA cocktail. The probes showed specific hybridization signal on a medium sized submetacentric chromosome identified as the Z-chromosome based on its morphology and G-banding pattern. After having confirmed the origin and purity of the preparation, the adaptors flanking the inserts were removed by *Sau*3AI digestion and column purification. Cloning was performed using equimolar ratios of the inserts to the vector ends (lambda ZAP Express, Stratagene). The original library consisted of a total of 8.48 X 10⁵ plaques representing about 14 chicken Z-chromosome equivalents. The final titer of the amplified library was 1.2 X 10¹² pfu/ml.

Thirty random plaques were selected and the inserts PCR-amplified using the T3/T7 priming sites flanking the inserts. The average insert size was about 1,000 bp (data not shown). This library was screened to identify microsatellite containing clones to increase the marker density of the chicken Z-chromosome genetic linkage map.

### Heterologous painting of turkey metaphase chromosomes:

The labeled chicken Z-chromosome-specific DNA fragments were used to perform FISH analysis on turkey metaphase chromosomes following the procedure described previously. Washes at the same stringency showed strong hybridization signals on a medium-sized submetacentric chromosome in turkey metaphases (data not shown). This chromosome was identified as the Z-chromosome homolog in the turkey. The obtained results indicate that the chicken and turkey Z-chromosome sequences are highly conserved. The red-legged partridge Z-chromosome has also been shown to be homologous to the chicken Z-chromosome (Dias el al, *Proc. of the XXIV Int*. *Cont. on Anim. Genet.,* Prague, Czech. p. 133 (July 23-24, 1994)). These results are similar to the FISH results obtained when the bovine X-chromosome painting probes were used on sheep and goat chromosomes (Ponce de León el al, *Proc. Natl. Acad. Sci., USA* (in press) (1996)) and with human X-chromosome probes on a wide range of mammalian species (Schertan el al, *Nat. Genet.,* 6:342-347 (1994)) indicating the high degree of sex chromosome conservation among all the mammalian species studied. Solinas-Toldo et al (*Genomics,* 27: 489-496 (1995)) have previously shown that human chromosome-specific painting probes could identify chromosomal segments in bovine that are homologous to specific human chromosomes. It is expected based on our results that chicken chromosome painting probes can similarly be used in closely and distantly related avian species to identify gross chromosomal rearrangements such as translocations and duplications that have occurred during avian evolution. Since the chicken Z-chromosome sequences are highly conserved in the turkey, the chicken Z-chromosome-specific microsatellite markers should be particularly useful for genetic mapping in turkey.

### Conclusions

Genetic and physical mapping of human and animal genomes has been greatly facilitated by the use of chromosome specific DNA libraries. Mapping with libraries specific to a chromosome or chromosomal region increases marker saturation by reducing the gaps resulting from a purely random shotgun approach. This study was undertaken to construct a genetic and physical map of microsatellites on the chicken Z chromosome. This chromosome is the fifth largest in the chicken genome, comprising about 8% of the total. Notwithstanding its size, very few microsatellites have been assigned to it. DNA originating from the chicken Z chromosome was previously isolated and reported. This was used to construct a small insert library in Lambda ZAP Express, representing 14 chromosome equivalents. This library was screened for microsatellites with an (AC) 12 oligo, and positive clones were isolated. Confirmation of the presence of the microsatellite, as well as its approximate location along the cloned fragment was accomplished by PCR amplification. Clones with adequate flanking regions were sequenced, and primers for 19 microsatellites were constructed. These primers were used to genotype individuals from the East Lansing Poultry Reference Population and a linkage map was constructed. Fourteen markers were scorable and polymorphic in this population. The resulting map contains 12 markers in two linkage groups spanning 90 Cm and two unlinked markers. The physical location of each marker was established by fluorescent *in situ* hybridization (FISH). Preliminary results with four markers allowed the assignment of one linkage group to the long arm of the Z chromosome, and one to the short arm.

The following nucleic acid sequences are microsatellite markers identified by the above methods. As discussed supra, these markers are useful for genetic mapping and for study of the sex chromosome structure in avian species. Also, such markers should enable the identification of genes encoding desirable traits, e.g., genes involved in growth rates, and for identifying sex-linked genotypes.

### EXAMPLE

The specific Gallus domesticus microsatellite markers identified are set forth below. As noted, these DNA markers will be useful for genetic mapping of domestic chicken as well as related avian species and for studies pertaining to evolution of the sex chromosome in avian species.

SEQUENCE 1 (43. Seq)
1 gatcactttc cctaatattc ttgtgtttct tgtttgttga cctgtaatgc
1 agttctgagt tttggaaagg aactaattaa gaccagagga gagataattt
101 tcttttatca aaaaacaaac aaacaaacaa aaaaacgaat tcttaccact
151 ttacaaaaat tttccatttt gaaggccagt acagccatag cattcatcta
201 ctttttgctt tggat

SEQUENCE 2 (71. Seq)
1 gatcaggtgg cctgtagtag acaacaacaa caatggggtg ccctttgttg
51 ccttagtctc taactcgcac ccacacacac tttcaagttg cttgtggcca
101 ttcttcaggg acagttcttc acaatctatt cctttcctga tgtagaaggc
151 gtcacctcct cccctcctgc ctcgtttgtc ccttctaaac tgcaggtatt
201 agtattgata gctaaggtca agtcatggga accatctcac caggtttcag
251 tgttggcaac tatgttatgc tttcttagga gcatggtggt tccaactctt
301 ccctgcttat ttcccaagct gtgtgtgatg gtaggatagc attcaagtgg
351 gaggagccta tcggcttttt ggaggtactc ctaaatccct gatattcccc
401 tgattcccgt acttcttcct tgccaagggc ccgccaatgc atagttcaat
451 ttctcatgca gacgctaagg aaaggtggac cc

SEQUENCE 3 (80 Seq.)

1 gatcgtatgt atttttttac ataggataga aaatggccaa taggaaataa

51 gacagtacag ctactaagaa agaaacacaa ttacacacac acacacacac

101 acacacacac acacatttga aaaacgcgct gcacagcagt gtgggtattt

151 tttcacaaga gagacacact ctacagtaca cagccagctc tactttgtcg

201 cacagtctca gtgtgtgttt gccaacagga cgcggttcac agggagatat

251 tgtcctcttg tgtgtgtgga gacacagaga cagag

SEQUENCE 4 (81. Seq)
1 gatcccctgg aggaagggca atggcaaccc actccagtat tcttgcctga
51 agaataccat ggtcagtttt gcctcctggg ctatagtcca tggggttgca
101 aagagtcagg catgactgag cgactctctc tctctctctc tctctctctc
151 acacacacac acacacacac acacacggcg tctctctctc tctctataca
201 tataggctgt gtgtctcgct attctcacat gagggaaact catatctagc
251 acgtggcaca aatattgttt gtggctctca caaaagacat gtgggcgcac
301 aaaggtcccc ccccggtgga tacancgcct tggtttttta taacccaagc
351 ctgtg

SEQUENCE 5 (131 Seq)
1 gatcacatat gtaaactagg gaattgcata ataagattaa atgtaggtgt
51 agaacgtggc atgaaggaag gtagaattag gtggtaccta tctcttctga
101 aacaaactga gaatcctact accaatcaac atattctaca taccacacac
151 acattttttc tcgagtaaaa tataaactaa tgagaaactt ccctag

SEQUENCE 6 (147. Seq)

1 gatcccaagc aacacatagn cagacaatca cacacacaca cacacacaca
51 cacacacaca cacacacaca cacatcctct ccccacaata catcccgaga
101 ggggggagag acactctctc tccctctcta taggggagac ccggagagct
151 ggctctgttg tctctctaca ccggacatac agtggagcac atctcacact
201 tgtgtctttg tctctctaca ccggacatac agtggagcac atctcacact
251 tgtgtctcta tctctccctg tccctgttga tccatctctc ttcacacatc
301 tctccagatc ttagcgctag agtctcctgt cttctctctg cgcaatttgt
351 gtgatagaga cacctgatat gttgtgtggg ggagacatct gtgtgtctct
401 gtgtcatccc agaggatttt tctctcccac acttagaggc cttctcaaga
451 gatgggaggt tttaatgggg tgtg

SEQUENCE 7 (166. Seq)
1 gatcattctt ctgtttccca ttctaatggg aattctccac acacacacac
51 acacacacac acacacacat cttcttcccc ttacatggaa aaaaatcctc
101 cacacccctg gacactgatt actctccctc ttcccagaga gagatc

SEQUENCE 8 (196. Seq)
1 gatcccctag agaagggaat ggctactcac tccagtattc ttgcctggag
51 aattccgtgg tcagaggagc ctggaaggct ataatccata gagtcgcaag
101 agtcagacag gactgagtga ctaacacaca catgcacaca cacacacaca
151 cacacacaca cttgctctag ggagaggcat agagatgtaa tctctcctaa
201 aatgggggtg gcgatggccc ctgcggccaa gtaatcgcca cacatgcgta
251 ttccccttaa gattgggtta ggcctcccct atgaggagag accagggaga
301 gaatgggctc tctctctctc tcactcccca accgagtaag tggtaaaaaa
351 ggttttcctg gattacaatt ttggtgttac agaattggaa aaaaatattt
401 ttggggctcc cccctcagtt ta

SEQUENCE 9 (199. Seq)
1 ctagcaaaaa cacccccaca agttatgaaa acaacggctt aatatagtaa
51 tgtgtgtgtg tgtgtgtgtg tgttgcacac cacagttttc tctgatactc
101 aaacctctct ctttctctac aggggccccc cataacacag cggctgagat
151 gtgtgacggg aaggcgtggc cttttacaca tttgtggtat ggtctgccaa
201 ggccccctat tgccccccac aactacggag atacactagg ggcgacccgc
251 aggcgcgcga cccccaggtg gggccccgag

SEQUENCE 10 (204. Seq)
1 ctttaggagg ttctctcgag taagcttttt ggatttcttt ggttcccaag
51 catcacatgg tacaggcagt cacacacaca cacatacaca cacacacaca
101 cacacacaca cactcctctc cccacaatac ataccgagag gggggagaga
151 cactctctct ccctctctat agggggagcc ccacagagct ggctctgttg
201 tctctctcca ccggacatac agtggagcac atctcacact tctgtctcta
251 tctctccctg cccctgtgac atccatctct cttcacacaa tctcacccag
301 gatcttagcg ctagagaccc cctgtccttc ttctcctggg gaaatttttt
351 gtggataaga gacacccgat atattggtgt gggggagaac atcttgtgag
401 gtctctgttg tgccatccca acaggaattt ttatctcccc cacaattaga
451 ggcccctcct caagagtgtg tgagggtt

SEQUENCE 11 (235. Seq)
1 gatcacagat gtatgtattt ttttacatag gatagaaaat ggacaatagg
51 aaataagaca gtacagctac taagaaagaa cccacattta cacacacaca
101 cacacacaca cacacacaca agtgtttaat ccgctgcaca gcattgtgga
151 catttttaca caagagagac acactctaca gtttgcgccc agctctag

SEQUENCE 12 (249. Seq.)
1 gatcattctt ctgtttccca ttctaatgga attctccaca cacacacaca
51 cacacacaca cacacactct tctttctcct gacatggaaa aatctccccc
101 acaccccggg acactgattt ctctccctct ccccaacact gtgagcaaga
151 ggagtttatt ttgtgtgtgt cactcttcca gggagagaga gatc

SEQUENCE 13 (258. Seq)
1 ctaggcatcg gttgggaggt ggtgagtaat tacttgtctg acattagtcc
51 tgtaacattg ggtgtgtgtg tgtgtgtgtg tgtgtattcc ccttgggaat
101 tggttttctc aaccacaagt tcttcttttt tttttttctc cccccttttc
151 ttctgaaaat aagtacttgg ggggtttccg ccccccccgg taaataaaat

SEQUENCE 14 (290. Seq)
1 ctagtggctc ccaagcaaca catagccaga caacacacac acacacacac
51 acacacacac acacacacac acacacactc ctctccccac aatacatccc
101 gagagggggg agagacactc tctctccctc tctatagcgg gagccccaca
151 gagctggctc tgctgtctct ctacaccgga catacagtgg agcacatctc
201 acattcgtgt ctctatctct ccctgcccct ggtgacatac atctctcttc
251 acacatctca ccaggtctga gcgctagagt ctcctgtctt ctctctgcgc
301 aatatttgtg atagagacat ctgatatatt gtgtgtggga gacatcttgt
351 gagtctctgt gtgcatccca gaggattttt atctccccac actag

SEQUENCE 15 (309. Seq)

1 gatccatgaa aactttccga gttgtattgt ctaggtgaaa acacacacaa

51 acacacacac acacacacac acacaacagg gagatgagtc ttgcaagaga

101 ataggggaga gttatgtcac caagtctggt gaggtatata gcgtataggg

151 agccaacatg tcagacatct gatgtgctaa gattaacatt ttattttatt

201 taatgtgtga gatctcatat agcggctctt cttatatatg acgtctcgca

251 atgtctcttt atgtgtgtta ttctctgagc ccctgggaga tatctgtcat

301 cagagagaag agacatacac atacaggggt tatatatttt ctccctgtgt

351 gtggagatgg agggtatttt ggacaagctc aacactcatt ggctcccaga

401 gagagaaaag gagcaactgt tgcacccggg gctctgtagc tgggatc

SEQUENCE 16 (341. Seq)

1 caattgggta catctacctg gtaccccacc cgggtggaaa atcgcatggg

51 cccgcggcgg ttctaggaag tactctcgag aagcttttgg gttctttggg

101 tcccaagcag cacatggaca ggcaatcaca cacacacaca cacacacaca

151 cacacacaca cacacacaca ctcctctccc cacaatacat cccgagaggg

201 gggagagtca ctctctctcc ctctctatag ggggcgcccc taagagctgg

251 ctctgttgtc tatctacacc gcacatacaa tggagcacaa ctcacactag

SEQUENCE 17 (398. Seq)
1 gatcaaagca tggaggtcat gccaggcact gaacaaaatg gtagagagtg
51 attctatgac tgactaagac ctcatgcaac aacaagtgaa gagtcacaac
101 tgcaaacaga agtacaactt agcaaatcct attttcagga aacactaaac
151 cgtaatactt gcacgatttt ttctttaata cagtaataat tcttttagaa
201 tttggatata tcttttaaga tacatatttg tctaaatacc aaggcaggat
251 atgagcataa aatagctaag gttagctatg gtgttatatt taagaagacc
301 acagagcaat aggagcatac ttttcttggg gtagaagggg cccttaaagg
351 tcacctag

SEQUENCE 18 (420. Seq)
1 ctagccacat cctataactc cactccacct ttaatcctga tttctgtgtc
51 tcttctctaa cctctatggc ctttctctaa agttccccaa tatcaacaat
101 ccttttcccc actgggacct ccagtttatt gattctacca tgtcactatc
151 catggtcaac cacttgtggt attataggat gtcgcgtgtg tgtgtgtgtg
201 tgtgtgcatg tgtgtgtgct tgggtgtcag agagttccaa tctgggggac
251 ctatggtttg taaacaacag gtctcttgcc aaggaagat

SEQUENCE 19 (435. Seq)
1 ctagcgctcg tgcccctgca gttcgacact cagtggctcc tccacacaca
51 cacacacaca cacatcaata tatatataga tagatagata gatagaggag
101 caatataagt ggcttctcta tttccagcat gttttgaaga gcataaactc
151 aacagagtat atataaatct gatgtgaccc atgtcatctg ctacagcatg
201 agagggggta gtgatc

## Claims

1. A Z-chromosomal marker DNA selected from the group consisting of Sequence I (43. Seq), Sequence 2 (71. Seq), Sequence 3 (80. Seq), Sequence 4 (81. Seq), Sequence 5 (131. Seq), Sequence 6 (147. Seq), Sequence 7 (166. Seq), Sequence 8 (196. Seq), Sequence 9 (199. Seq), Sequence 10 (204. Seq), Sequence 11 (235. Seq), Sequence 12 (249. Seq), Sequence 13 (258. Seq), Sequence 14 (290. Seq), Sequence 15 (309. Seq), Sequence 16 (341. Seq), Sequence 17 (398. Seq), Sequence 18 (420. Seq), and Sequence 19 (435. Seq).

2. A Z-chromosomal DNA library that contains at least one DNA sequence according to Claim 1.

3. A method of using at least one Z-chromosomal DNA according to Claim 1 for genetic mapping.

4. The method of Claim 3, wherein the genetic mapping is effected to construct a Z-chromosome specific DNA map.

5. The method of Claim 3, wherein the Z-chromosome DNA map is that of an avian species selected from the group consisting of chicken, turkey, partridge, duck, guinea hen, and goose.

6. The method of Claim 4, which is used to identify gross chromosomal rearrangements.

7. The method of Claim 6, wherein said chromosomal rearrangement comprises a translocation, deletion or duplication.

8. Use of a Z-chromosomal marker DNA according to claim 1 as a probe to identify a Z-chromosomal homolog in a related avian species.

9. Use according to claim 8 where the related avian species is a turkey.

## Patentansprüche

1. Z-Chromosomale Marker-DNS, ausgewählt aus der Gruppe, bestehend aus Sequenz I (43. Seq), Sequenz 2 (71. Seq), Squenz 3 (80. Seq), Sequenz 4 (81. Seq), Sequenz 5 (131. Seq), Sequenz 6 (147. Seq), Sequenz 7 (166. Seq), Sequenz 8 (196. Seq), Sequenz 9 (199. Seq), Sequenz 10 (204. Seq), Sequenz 11 (235. Seq), Sequenz 12 (249. Seq), Sequenz 13 (258. Seq), Sequenz 14 (290. Seq.), Sequenz 15 (309. Seq), Sequenz 16 (341. Seq), Sequenz 17 (398. Seq), Sequenz 18 (420. Seq) und Sequenz 19 (435. Seq).

2. Z-chromosomale DNS-Datenbank, die mindestens eine DNS-Sequenz gemäß Anspruch 1 enthält.

3. Verfahren zur Verwendung mindestens einer Z-chromosomalen DNS gemäß Anspruch 1 für die Genkartierung.

4. Verfahren nach Anspruch 3, worin die Genkartierung ausgeführt wird, um eine Z-Chromosomen-spezifische DNS-Karte zu konstruieren.

5. Verfahren nach Anspruch 3, worin die Z-Chromosomen-DNS-Karte diejenige einer Vogel-Spezies ist, ausgewählt aus der Gruppe, bestehend aus Huhn, Truthahn, Rebhuhn, Ente, Perlhuhn und Gans.

6. Verfahren nach Anspruch 4, das zur Identifikation von groben chromosomalen Umordnungen verwendet wird.

7. Verfahren nach Anspruch 6, worin die chromosomale Umordnung eine Translokation, Deletion oder Duplikation umfasst.

8. Verwendung einer Z-chromosomalen Marker-DNS gemäß Anspruch 1 als Sonde zum Identifizieren eines Z-Chromosomalen Homologen in einer verwandten Vogel-Spezies.

9. Verwendung gemäß Anspruch 8, worin die verwandte Vogel-Spezies ein Truthahn ist.

## Revendications

1. ADN marqueur de chromosome Z, choisi dans l'ensemble constitué par la Séquence 1 (43: Seq), la Séquence 2 (71. Seq), la Séquence 3 (80. Seq), la Séquence 4 (81. Seq), la Séquence 5 (131. Seq), la Séquence 6 (147. Seq), la Séquence 7 (166. Seq), la Séquence 8 (196. Seq), la Séquence 9 (199. Seq), la Séquence 10 (204. Seq), la Séquence 11 (235. Seq), la Séquence 12 (249. Seq), la Séquence 13 (258. Seq), la Séquence 14 (290. Seq), la Séquence 15 (309. Seq), la Séquence 16 (341. Seq), la Séquence 17 (398. Seq), la Séquence 18 (420. Seq), et la Séquence 19 (435. Seq).

2. Bibliothèque d'ADN de chromosome Z, qui contient au moins une séquence d'ADN conforme à la revendication 1.

3. Procédé où l'on emploie au moins un ADN marqueur de chromosome Z conforme à la revendication 1 pour faire de la cartographie génétique.

4. Procédé conforme à la revendication 3, dans lequel on fait de la cartographie génétique pour établir une carte d'ADN de chromosome Z spécifique.

5. Procédé conforme à la revendication 3, dans lequel la carte d'ADN de chromosome Z est celle d'une espèce d'oiseau choisie dans l'ensemble formé par le poulet, le dindon, la perdrix, le canard, la pintade et l'oie.

6. Procédé conforme à la revendication 4, employé pour identifier d'importants réarrangements chromosomiques.

7. Procédé conforme à la revendication 6, dans lequel ledit réarrangement chromosomique comporte une translocation, une délétion ou une duplication.

8. Emploi d'un ADN marqueur de chromosome Z, conforme à la revendication 1, en guise de sonde pour identifier un homologue de chromosome Z chez une espèce d'oiseau apparentée.

9. Emploi conforme à la revendication 8, dans lequel l'espèce d'oiseau apparentée est le dindon.
